# EUROPÄISCHE PATENTSCHRIFT

(11) **EP 0 475 903 B1**
(45) Veröffentlichungstag und Bekanntmachung des Hinweises auf die Patenterteilung: **24.04.1996**
(21) Anmeldenummer: 91810707.9
(22) Anmeldetag: 04.09.1991
(51) Int. Cl.: C07D 309/10, G03F 7/004

(54) **Säurelabile Lösungsinhibitoren und darauf basierende positiv und negativ arbeitende strahlungsempfindliche Zusammensetzung**
Acid labile solution inhibitors and positively and negatively working radiation sensitive composition based on them
Inhibiteurs de solution labiles aux acides et composition sensible aux rayonnements à effec positif et negatif à base de ceux-ci

(30) Priorität: 13.09.1990 CH 2971/90
(43) Veröffentlichungstag der Anmeldung: 18.03.1992
(73) Patentinhaber: OCG Microelectronic Materials Inc., West Paterson New Jersey 07424 (US)
(72) Erfinder: Schädeli, Ulrich, Dr., CH-1763 Granges-Paccot (CH)
(74) Vertreter: Klunker . Schmitt-Nilson . Hirsch

(56) Entgegenhaltungen:
- US-A- 4 908 381
- CHEMICAL ABSTRACTS, vol. 73, no. 19, 9. November 1970, Columbus, Ohio, US; Seiten 356-357, abstract no. 98 735y, V.G. KOZYREV et al.: 'Aryloxydihydropyrans. V. Effect of the nature of the substituent on acetal formation'

## Beschreibung

Die vorliegende Erfindung betrifft neue nichtpolymere Verbindungen, die mindestens ein aromatisches Ringsystem mit einem oder mehreren speziellen Tetrahydropyranyloxysubstituenten aufweisen, Zusammensetzungen, die diese Verbindungen enthalten, Verfahrenen zur Erzeugung positiver und negativer Abbildungen mit Hilfe solcher Zusammensetzungen und die unter Verwendung dieser Verfahren herstellbaren Produkte.

Seit langem sind Photoresistsysteme bekannt und werden z.B. zur Herstellung von Druckplatten, silberlosen photographischen Filmen, Platinen und integrierten Schaltungen verwendet.

Ein spezieller Typ solcher Photoresists besteht aus einer Verbindung A, die bei Bestrahlung unter Bildung einer Protonen- oder Lewissäure reagiert, und zumindest einer weiteren Verbindungen B, die in irgendeinem ausgewählten Lösungsmittel nicht oder kaum löslich ist und auch eine Lösungstendenz anderer gegebenenfalls noch in dem Photoresist vorhandener Verbindungen in diesem Lösungsmittel verhindert oder stark senkt (Lösungsinhibitor). Die Verbindung B reagiert mit der bei Bestrahlung von Verbindung A gebildeten Säure. Dabei wird die Verbindung B chemisch so verändert, dass sie in dem gewählten Lösungsmittel löslich wird und folglich auch das Resistsystem an den bestrahlten Stellen im gewählten Lösungsmittel gelöst werden kann.

Da die bei der Bestrahlung freigesetzte Säure die Abreaktion der Verbindung B katalysiert, sind nur geringe Mengen Säure nötig, die die Verbindung B bei Erwärmung vollständig in die lösliche Form überführen können (Chemische Amplifikation).

Positiv arbeitende Photolacksysteme, die nach dem oben geschilderten Prinzip arbeiten, sind beispielsweise in der US-A-3,779,778 beschrieben. Als Lösungsinhibitoren werden darin u.a. auch Arylverbindungen erwähnt, die als Substituenten Tetrahydropyranyloxygruppen aufweisen, die gegebenenfalls ihrerseits alkyl- oder phenylsubstituiert sein können. Die geschilderten Photolacksysteme sind für Strahlung von 300 bis 700 nm Wellenlänge geeignet. Die während der Belichtung gebildete Säure führt unter katalytischer Abspaltung der Tetrahydropyrangruppen zur Bildung der Hydroxyarylverbindungen, die im Gegensatz zum nicht reagierten Lösungsinhibitor in wässrigen alkalischen Lösungen relativ gut löslich sind, so dass die Lackschicht mit solchen Lösungen als Entwickler an den belichteten Stellen gelöst werden kann.

Insbesondere bei der Herstellung hochintegrierter Schaltungen ist es aber nötig, mit kurzwelligerer Strahlung zu arbeiten, als sie in der vorgenannten US-Patentschrift für die dort erwähnten Photolacksysteme beschrieben ist. Oftmals wird dabei im Tief-UV-Bereich (ca. 200-300 nm Wellenlänge) gearbeitet, damit noch genügend feine Strukturen abgebildet werden können (Tief-UV-Mikrolithographie). Dabei müssen die Photoresistschichten viel empfindlicher sein als bei Verwendung von Strahlung mit Wellenlängen oberhalb 300 nm. Die für die Tief-UV-Mikrolithographie häufig eingesetzten Quecksilberdampflampen weisen nämlich nur einen sehr geringen Anteil Strahlung mit einer Wellenlänge von 254 nm auf, bezogen auf den Anteil an längerwelliger Strahlung (365 nm und 436 nm). Für die Tief-UV-Mikrolithographie wurden ebenfalls schon Photoresistsysteme mit einem strahlungsempfindlichen Säuregenerator und einem Lösungsinhibitor vorgeschlagen (Dennis R. McKean, Scott A. MacDonald, Nicholas J. Clecak and C. Grant Willson, "Novolak based deep-UV resists", SPIE, Vol. 920 Advances in Resist Technology and Processing V, p. 60-66 (1988)). Als Lösungsinhibitor wird ein tert.-Butylcarbonatderivat des Bisphenols A verwendet, das zusammen mit Triphenylsulfoniumhexafluoroantimonat und einem Novolak-Harz einen positiv arbeitenden Photoresist bildet, der Strukturen bis hinunter zu 1 µm aufzulösen vermag.

In der US-A-4,908,381 und in CHEMICAL ABSTRACTS, Vol 73, no. 19 (9. Nov. 1970), Columbus, Ohio, US; Seiten 356, 357, abstract. no. 98 732 sind bereits Verbindungen beschrieben, die einen Tetrahydropyranyloxysubstituenten gemäss der unten angegebenen Formel I aufweisen, wobei dieser Substituent auch an ein aromatisches Ringsystem gebunden sein kann. Die US-A-4,908,381 beschreibt weiterhin die antimikrobielle Wirkung dieser Verbindungen.

Es wurde nun gefunden, dass sich diese und andere neuartige Verbindungen, die in bestimmter Weise substituierte Tetrahydropyranyloxygruppen an Aromaten gebunden, enthalten, besonders gut als Lösungsinhibitoren sowohl für positiv als auch für negativ arbeitende Photoresists eignen, insbesondere wenn hohe Anforderungen an die Auflösung feinster Strukturen gestellt werden.

Gegenstand der Erfindung sind daher einerseits nichtpolymere Verbindungen, wobei dieser Begriff auch Oligomere eines Polymerisationsgrades unter 10 umfasst und die Verbindungen mindestens ein aromatisches Ringsystem mit einem oder mehreren Tetrahydropyranyloxysubstituenten der Formel I aufweisen und wobei die Verbindungen insgesamt mindestens zwei dieser Substituenten enthalten: 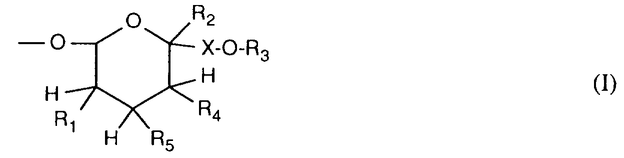 wobei
R₁ Wasserstoff, Halogen, Alkyl, Cycloalkyl, Aryl, Alkoxy oder Aryloxy,
R₂ Wasserstoff, Alkyl, Cycloalkyl oder Aryl,
R₃ einen gesättigten oder ungesättigten Kohlenwasserstoffrest,
R₄, R₅ unabhängig voneinander Wasserstoff, Halogen, Alkyl, Alkoxy oder Aryloxy und
X eine direkte Einfachbindung, eine Methylen- oder Ethylenbrücke bedeuten können.

Bedeuten R₁ und/oder R₂ in Formel I Alkyl, so kann es sich um kettenförmige, d.h. geradkettige oder verzweigte Alkylreste handeln. Insbesondere dann, wenn nicht zu niedrige Schmelzpunkte für die erfindungsgemässen Verbindungen gewünscht werden, was beispielsweise für Photoresistanwendungen vorteilhaft ist, sollten die Alkylreste nicht zu lang sein. Gut geeignet sind in diesem Fall Alkylreste mit 1 bis 5 Kohlenstoffatomen. Kurze Kohlenstoffreste fördern auch die Löslichkeit der Verbindungen in mehr polaren Lösungsmitteln, was ebenfalls in gewissen Fällen von Vorteil sein kann. Beispiele für solches Alkyl sind etwa Methyl, Ethyl, n-Propyl, Isopropyl, n-Butyl, sek.-Butyl, tert.-Butyl, n-Pentyl, Isoamyl. Ganz besonders bevorzugt wird Methyl als Alkylrest für R₁ und/oder R₂.

Als Cycloalkylreste für R₁ und/oder R₂ kommen z.B. solche mit 1 bis 8 Kohlenstoffatomen in Frage, wie Cyclopentyl-, methylsubstituierte Cyclopentyl-, Cyclohexyl- oder Cycloheptylreste.

Bedeuten R₁ und/oder R₂ dagegen Aryl, so handelt es sich besonders bevorzugt um Phenyl oder substituiertes Phenyl. Geeignete Substituenten für Phenyl sind z.B. Alkyl und Alkoxy, vorzugsweise mit je 1 bis 4 Kohlenstoffatomen, oder Halogen. Grössere aromatische Systeme, z.B. Naphthyl und noch grössere, rufen bei den erfindungsgemässen Verbindungen bereits eine relativ starke Eigenabsorption im UV und/oder im sichtbaren Bereich hervor. Insbesondere für die Anwendung als Lösungsinhibitor für im Tief-UV empfindliche Photoresists sind diese Verbindungen weniger geeignet. Ein Anwendungsbereich für solche Verbindungen wäre aber z.B. der Zusatz zu röntgenstrahlenempfindlichen Photoresists.

Als Alkoxyrest kann R₁ bevorzugt einen Alkoxyrest mit 1 bis 5 Kohlenstoffatomen darstellen, beispielsweise Methoxy-, Ethoxy-, n-Propoxy, Isopropoxy- oder einen der verschiedenen Butoxy- oder Pentoxyreste.

Bedeutet R₁ Aryloxy, kommt vor allem der Phenoxy- oder ein substituierter Phenoxyrest in Frage und, wenn die Eigenabsorption der erfindungsgemässen Verbindungen nicht stört, auch ein entsprechender Rest grösserer aromatischer Systeme wie z.B. Naphthyloxy. Als Substituenten für den Phenoxyrest kommen z.B. Alkyl oder Alkoxy mit vorzugsweise je 1 bis 4 Kohlenstoffatomen oder Halogen in Frage.

Stellt R₁ ein Halogenatom dar, so sind Fluor, insbesondere aber Chlor und Brom und auch Jod geeignet.

R₃ kann ein geradkettiger oder verzweigtkettiger Kohlenwasserstoffrest sein, ebenso ein cyclischer. Die Zahl der Kohlenstoffatome kann beispielsweise 1 bis 20 betragen. Bevorzugt sind besonders kürzere acyclische Reste mit 1 bis 8 Kohlenstoffatomen, z.B. Methyl, Ethyl, n-Propyl, Isopropyl, n-Butyl, sek.-Butyl, tert.-Butyl, i-Butyl, n-Pentyl, i-Pentyl, Isoamyl, n-Hexyl, n-Octyl oder Isooctyl oder die entsprechenden einfach oder mehrfach ungesättigten Alkenyl- oder Alkinylreste, also z.B. Vinyl, Ethinyl, 1-Propenyl, Allyl, 1-Methyvinyl, Butenyl, Butadienyl, Butinyl, Pentenyl, Pentadienyl, Heptadienyl u.s.w. und Arylreste. Beispiele für Arylreste sind Phenyl, substituiertes Phenyl und Naphthyl. Besonders geeignete Substituenten für Phenyl sind wiederum Alkyl und Alkoxy mit je 1 bis 4 Kohlenstoffatomen oder Halogen. Beispiele für geeignete nichtaromatische cyclische Kohlenwasserstoffe R₃ sind Cyclopentyl, Cyclopentenyl, Cyclohexyl, Cyclohexenyl oder Cyclohexadienyl. Ganz besonders bevorzugt werden aber Alkyketten mit 1 bis 5 Kohlenstoffatomen, insbesondere Methyl, Ethyl und i-Butyl, sowie Phenyl. Neben diesen sind für die Anwendung als Lösungsinhibitor in positiven Photoresists oftmals solche Reste R₃ besonders geeignet die besonders stabile Carboniumionen auszubilden vermögen, z.B. der Allylrest oder andere allylisch ungesättigte Reste R₃, da sich in diesen Fällen auch die Etherbindung bei R₃ relativ leicht mit Säure spalten lässt, so dass die aufgrund der Belichtung aus dem Lösungsinhibitor abgespaltene Pyranylgruppe leicht weiter in kleinere Bestandteile aufgespalten werden kann, wodurch sich die Löslichkeit der belichteten Teile der Resistbeschichtung in den üblichen Entwicklern weiter verbessert.

R₄ und/oder R₅ als Alkyl- oder Alkoxyreste weisen bevorzugtermassen ebenfalls 1 bis 5 Kohlenstoffatome auf. Als Beispiele wird auf die oben für R₁ bzw. R₂ genannten verwiesen. Bedeuten R₄,R₅ Aryloxy so sind wiederum Phenoxy bzw. substituiertes Phenoxy bevorzugt. Als Substituenten für den Phenoxyrest kommen z.B. Alkyl und Alkoxy mit vorzugsweise je 1 bis 4 Kohlenstofftomen oder Halogen in Betracht. Für R₄, R₅ als Halogen gilt das bereits für R₁ in der Bedeutung Halogen gesagte. Besonders bevorzugt ist es, wenn R₄ und R₅ einen Methylrest oder Wasserstoff darstellen.

Bevorzugt werden erfindungsgemässe Verbindungen, wobei in der Formel I der Rest R₁ Wasserstoff, Halogen, C₁-C₅-Alkyl, Phenyl, substituiertes Phenyl, C₁-C₅-Alkoxy, Phenoxy oder substituiertes Phenoxy, R₂ Wasserstoff, C₁-C₅-Alkyl oder Phenyl, R₃ einen gesättigten oder ungesättigten Kohlenwasserstoffrest mit 1 bis 20 Kohlenstoffatomen und R₄, R₅ unabhängig voneinander Wasserstoff, Halogen, C₁-C₅-Alkyl, C₁-C₅-Alkoxy, Phenoxy oder substituiertes Phenoxy bedeuten können.

Am meisen bevorzugt werden schliesslich die Verbindungen, in denen R₁ Wasserstoff, Methyl oder Phenyl ist, R₂, R₄, R₅ alle Wasserstoff sind und R₃ für einen gesättigten oder ungesättigten acyclischen Kohlenwasserstoffrest mit 1 bis 8 Kohlenstoffatomen oder für ein Phenyl steht, speziell dann, wenn auch R₁ Wasserstoff ist.

Bevorzugte Typen der erfindungsgemässen Verbindungen sind auch die, in denen X für eine direkte Bindung steht, da sie besonders gut zugänglich sind und auch die Säurespaltung der Etherbrücke zwischen dem Tetrahydropyranring und dem Rest R₃ in diesem Fall besonders leicht verläuft.

Bei den erfindungsgemässen Verbindungen handelt es sich um nichtpolymere Verbindungen. Der Begriff "nichtpolymer" soll jedoch Oligomere eines Polymerisationsgrades unter ca. 10 nicht ausschliessen. Die erfindungsgemässen Verbindungen weisen im allgemeinen maximal ca. 100, insbesondere maximal ca. 50, Gerüstatome auf, wobei als Gerüstatome alle Kohlenstoff- und Heteroatome gezählt werden, jedoch die Tetrahydropyranyloxygruppen nicht mitgerechnet sind. Als Heteroatome kommen insbesondere N, 0, S, Halogene, insbesondere F, Cl und Br, sowie Si in Frage. Mindestens 75% der Gerüstatome der erfindungsgemässen Verbindungen sollen zu aromatischen Systemen gehören. Die erfindungsgemässen Verbindungen enthalten zweckmässig mindestens zwei Tetrahydropyranyloxysubstituenten der Formel I, ausserdem soll die Gesamtzahl der Substituenten der Formel I, die die erfindungsgemässen Verbindungen aufweisen, mindestens so gross sein, dass auf zwei aromatische Ringe in den Verbindungen mindestens ein Substituent der Formel I kommt, wobei mit aromatischen Ringen jeweils die einzelnen aromatischen Ringe eines aromatischen Systems gemeint sind.

Für Photoresistanwendungen enthalten die erfindungsgemässen Verbindungen jedoch zweckinässig mehr als zwei Tetrahydropyranyloxysubstituenten. Dabei kann an einen aromatischen Ring nicht nur eine einzige Tetrahydropyranyloxygruppe der Formel I gebunden sein, sondern auch zwei, drei oder unter Umständen noch mehr. Die Obergrenze wird aber im allgemeinen durch die sterischen Verhältnisse im Molekül auf zwei bis drei Gruppen der Formel I pro aromatischem Ring begrenzt.

Die aromatischen Systeme der erfindungsgemässen Verbindungen weisen bevorzugt 6 bis 14 Ringkohlenstoffatome, ganz besonders bevorzugt 6 Ringkohlenstoffatome, auf. Erfindungsgemässen Verbindungen, die ausschliesslich aromatische Ringsysteme mit 6 Ringkohlenstoffatomen aufweisen, sind, insbesondere wenn die aromatischen Ringsysteme nicht konjugiert sind, besonders pot in Fällen geeignet, wo eine geringe Absorption im UV- und sichtbaren Bereich erwünscht ist, z. B. also zur Verwendung als Lösungsinhibitoren in Photoresists für das UV-Gebiet.

Wertvolle Typen erfindungsgemässer Verbindungen sind z. B. die Verbindungen der Formel II 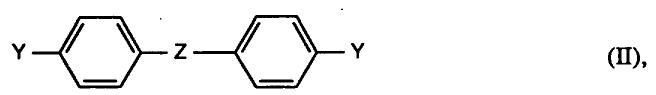 wobei Y jeweils einen Tetrahydropyranyloxysubstituenten der Formel I und Z entweder eine direkte Einfachbindung oder eine der folgenden Gruppen bedeuten kann: -S-; -O-; -SO-; -SO₂-; -CO-; -C(R₆)(R₇)-, wobei R₆ Wasserstoff, Methyl oder Aryl sowie R₇ Wasserstoff oder Methyl darstellen können. Besonders bevorzugte zweiwertige Reste -C(R₆)(R₇)- sind -CH₂-; -C(CH₃)₂- und -C(CH₃)(Ph)-.

Besonders bevorzugt sind auch die Verbindungen mit der Formel III 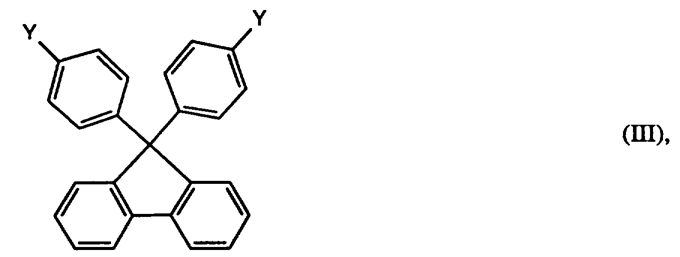 worin Y für einen der beschriebenen erfindungsgemässen Tetrahydropyranyloxysubstituenten steht und die sich vom 9,9-Bis(4-hydroxy)phenylfluoren ableiten.

Besonders bevorzugt sind Verbindungen der Formeln II oder III, wenn sie Tetrahydropyranyloxysubstituenten der Formel I aufweisen, wobei R₁ Wassertoff, Methyl oder Phenyl, R₂, R₄, R₅ alle Wasserstoff und R₃ einen gesättigten oder ungesättigten acyclischen Kohlenwasserstoffrest mit 1 bis 8 Kohlenstoffatomen oder Phenyl bedeuten können, insbesondere die Verbindungen der folgenden Formel 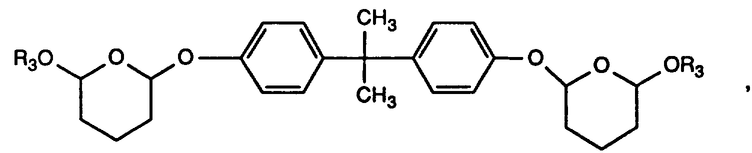 worin R₃ Methyl, Ethyl, i-Butyl oder Phenyl bedeutet, und die Verbindungen der folgenden Formel 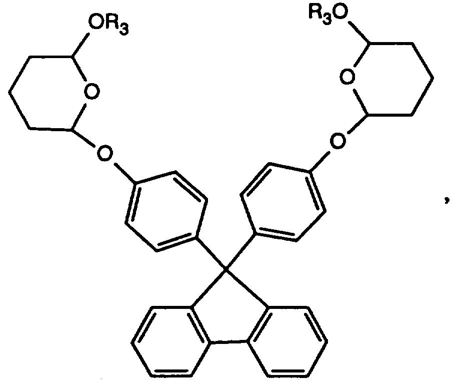 worin R₃ i-Butyl oder Phenyl bedeutet.

Selbstverständlich können die aromatischen Ringsysteme der erfindungsgemässen Verbindungen naben den oben bescriebenen Tetrahydropyranyloxysubstituenten noch übliche andersartige Substituenten aufweisen, insbesondere C₁-C₅-Alkyl, C₁-F₅-Alkoxy, Halogen und ähnliche. Unzweckmässig sind allerdings solche Substituenten, die die Löslichkeit der erfindungsgemässen Verbindungen in wässrigen oder wässrig-alkalischen Lösungen stark erhöhen können, z. B. -COOH oder leicht lösliche -COOMe-Gruppen (Me=Metall).

Die erfindungsgemässen Verbindungen lassen sich z.B. in an sich bekannter Weise durch eine Additionsreaktion von ca. stöchiometrischen Mengen einer aromatischen Hydroxylverbindung und geeigneter 3,4-Dihydro-2H-pyrane darstellen, wobei gewöhnlich unter wasserfreien Bedingungen und unter Katalyse von starken Säuren, wie Chlorwasserstoffsäure, Bortrifluorid oder p-Toluolsulfonsäure, gearbeitet wird. Dieses Darstellungsverfahren ist z.B. in Journal of the American Chemical Society, 70, 4187-4189 (1948) beschrieben.

Besonders gut geeignet sind aromatische Polyhydroxylverbindungen, die keine Hydroxylgruppen an benachbarten Kohlenstoffatomen aufweisen, z. B. 1,3-Dihydroxybenzol, 1,4-Dihydroxybenzol, 1,3,5-Trihydroxybenzol, 2,2'-Dihydroxybiphenyl, 2,2',4,4'-Tetrahydroxybiphenyl, 4,4'-Isopropylidendiphenol, 4,4'-Oxydiphenol, 4,4'-Sulfonyldiphenol, 2,4-Oxydiphenol, 2,4'-Sulfonyldiphenol, 1,1-Di-(4-hydroxyphenyl)-1-phenylmethan, 1,1,1-Tri-(4-hydroxyphenyl)methan, 1-(3,5-Dihydroxyphenyl)-1,1-diphenylmethan, 1,1-Di-(3,5-dihydroxyphenyl)-1-phenylmethan, 1,1 -Di-(4-hydroxyphenyl)-1-phenylethan, 1,1,1 -Tri-(4-hydroxyphenyl)ethan oder 1,3,5-Tri-(4-hydroxyphenyl)benzol.

Geeignete 2-Alkoxy-3,4-dihydro-2H-pyrane, bzw. entsprechende 2-Alkenyloxy- oder Alkinyloxydihydropyrane und 2-Aryloxy-3,4-dihydro-2H-pyrane sowie 2-Alkoxyalkyl-3,4-dihydro-2H-pyrane bzw. die entsprechenden 2-Alkenyloxyalkyl- und 2-Alkinyloxyalkylderivate sowie die 2-Aryloxyalkyl-3,4-dihydro-2H-pyrane sind durch Cycloaddition von Acrolein und dem entsprechenden Vinylether bei erhöhter Temperatur im Autoklaven zugänglich, wie z.B. in US-A-2,514,168 beschrieben. Weitere Beispiele findet man u.a. in Beilstein, Ergänzungswerk III, IV, Vol. 17, p. 1182-1195. Teilweise sind für die Herstellung erfindungsgemässer Verbindungen geeignete 3,4-Dihydropyrane auch kommerziell erhältlich.

Die erfindungsgemässen Verbindung können ebenso wie entsprechende nichtpolymere Verbindung mit nur einem Tetrahydropyranyloxysubstituenten der Formel I in Photoresistsystemen als Lösungsinhibitor eingesetzt werden. Die Erfindung betrifft daher auch eine Zusammensetzung, enthaltend eine dieser Verbindungen, ferner eine Verbindung, die unter Einwirkung aktinischer Strahlung Säure bildet, und ein Bindemittel. Je nach Art der Substituenten an den Tetrahydropyranyloxygruppen der erfindungsgemässen Verbindungen lässt sich die Polarität der Verbindungen und damit ihr Inhibierungsverhalten in weiten Grenzen regeln und den restlichen Komponenten des Resistsystems, insbesondere dem Bindemittel, weitgehend anpassen. Beispielsweise kann durch Verwendung relativ unpolare Substituenten aufweisender erfindungsgemässer Verbindungen als Lösungsinhibitoren eine noch genügende Lösungsinhibierung von unbelichteten Zonen des Resistfilms auch bei Anwesenheit eines in wässrig-alkalischen Entwicklern stark löslichen Bindemittels, wie es z. B. Poly-(4-hydroxystyrol) ist, erreicht werden.

Die erfindungsgemässen Zusammensetzungen enthalten in der Regel 5-50 Gew.-% Aryltetrahydropyranyletherverbindungen, bezogen auf die Gesamtmenge der Zusammensetzung an nicht flüchtigen Bestandteilen. Bevorzugt verwendet man 10-40 Gew.-% der Verbindung.

Als strahlungsempfindliche Komponenten, die bei Lichteinwirkung Säure bilden bzw. abspalten, sind eine grosse Anzahl von Verbindungen bekannt. Dazu zählen beispielsweise Diazoniumsalze, wie sie in der Diazotypie verwendet werden, o-Chinondiazide, wie sie in bekannten positiv arbeitenen Kopiermassen verwendet werden, oder auch Halogenverbindungen, die bei Bestrahlung Halogenwasserstoffsäure bilden. Verbindungen dieses Typs sind beispielsweise in den US-A-3,515,552, 3,536,489 oder 3,779,778, sowie in den DE-A-2,718,259, 2,243,621 oder 2,610,842 beschrieben.

Als strahlungsempfindliche Komponenten der erfindungsgemässen Zusammensetzung eignen sich aber auch kationische Photoinitiatoren aus der Gruppe der Iodonium- oder Sulfoniumsalze. Solche Verbindungen sind beispielsweise in "UV-Curing, Science and Technology" (Editor: S.P. Pappas, Technology Marketing Corp., 642 Westover Road, Stanford, Connecticut, USA) beschrieben.

Insbesondere sind auch Diaryliodosyl-Salze einsetzbar. Solche Verbindungen sind beispielsweise in der EP-A-106,797 beschrieben.

Ferner können Sulfoxoniumsalze als strahlungsempfindliche Verbindungen verwendet werden. Solche Salze sind beispielsweise in der EP-B-35,969 oder in den EP-A-44,274 bzw. 54,509 beschrieben. Insbesondere zu erwähnen sind aliphatische Sulfoxoniumsalze, die im tiefen UV-Bereich absorbieren, wie beispielsweise in der EP-A- 164,314 beschrieben.

Insbesondere lassen sich auch Verbindungen einsetzen, die bei Besatrahlung mit aktinischem Licht Sulfonsäwen freisetzen. Solche Verbindungen sind an sich bekannt und beispielsweise in der GB-A 2,120,263, den EP-A 84,515, 37,152 oder 58,638 bzw. in den US-A-4,258,121 oder 4,371,605 beschrieben.

Werden als strählungsempfindliche säureabspaltende Komponenten Salze eingesetzt, so sind diese vorzugsweise in organischen Lösungsmitteln löslich. Besonders bevorzugt handelt es sich bei diesen Salzen um Abscheidungsprodukte mit komplexen Säuren, beispielsweise von Borfluorwasserstoffsäure oder Hexafluorphosphorsäure.

Die Menge der srtrahlungsempfindlichen Komponente der erfindungsgemässen Zusammensetzungen kann je nach Natur und Zusammensetzung des strahlungsempfindlichen Gemisches in weiten Grenzen variiert werden. Günstige Ergebnisse werden erzielt, bei etwa 0, 1 bis 20 Gew.-% an strahlungsempfindlicher Komponente, bezogen auf den Gesamtgehalt der Zusammensetzung an nicht flüchtigen Bestandteilen. Vorzugsweise setzt man 0,2 bis 10 Gew.-% Säurespender ein.

Den erfindungsgemässen Photoresistzusammensetzungen muss auch ein Bindemittel zugesetzt werden. Die Menge des Bindemittels kann 30-90 Gew.-%, vorzugsweise 60-90 Gew.-% betragen, bezogen auf den Gesamtgehalt der Zusammensetzung an Bindemittel, strahlungsempfindlicher säureabspaltender Komponente und erfindungsgemässer Verbindung. Vorzugsweise verwendet man als Bindemittel eine besonders gut alkalilösliche Substanz.

Gut geeignet sind Bindemmittel auf Basis phenolischer Verbindungen, beispielsweise Novolake, die sich von einem Aldehyd, vorzugsweise Acetaldehyd oder Furfuraldehyd, besonders jedoch von Formaldehyd, und einem Phenol ableiten. Die phenolische Komponente dieser Bindemittel ist vorzugsweise Phenol selbst, oder auch halogeniertes Phenöl, beispielsweise substituiert mit ein bis zwei Chloratomen, vorzugsweise p-Chlorphenol, oder sie ist ein durch ein- bis zwei C₁-C₉-Alkylgruppen substituiertes Phenol, beispielsweise o-, m- oder p-Kresol, ein Xylenol, p.-tert.Butylphenol oder p-Nonylphenol. Es kann sich bei der Phenolkomponente der bevorzugten Novolake aber auch um p-Phenylphenol, Resorcin, Bis-(4-hydroxyphenyl)-methan oder 2,2-Bis-(4-hydroxyphenyl)-propan handeln.

Ein Teil der phenolischen Hydroxygruppen dieser Novolake kann gegebenenfalls durch Umsetzung mit Chloressigsäure, Isocyanaten, Epoxiden, Carbonsäureanhydriden, Carbonsäurechloriden oder Sulfonsäurechloriden modifiziert sein.

Ebenfalls gut geeignet als Bindemittel sind Poly-(4-hydroxystyrole).

Die erfindungsgemässe Zusammensetzung kann weitere übliche Zusatzstoffe enthalten, wie z.B. Stabilisatoren, Pigmente, Farbstoffe, Füllstoffe, Haftvermittler, Verlaufmittel, Netzmittel und Weichmacher. Ferner können die Zusammensetzungen zur Applikation in geeigneten Lösungsmitteln gelöst werden.

Die erfindungsgemässen Zusammensetzungen eignen sich hervorragend als Beschichtungsmittel für Substrate aller Art, z.B. Holz, Textilien, Papier, Keramik , Glas, Kunststoffe, wie Polyester, Polyethylenterephthalat, Polyolefine oder Celluloseacetat, insbesondere in Form von Filmen, sowie von Metallen, wie AI, Cu, Ni, Fe, Zn, Mg oder Co, und von Si oder SiO₂, bei denen durch bildmässiges Belichten eine Abbildung aufgebracht werden soll.

Die oben beschriebenen Zusammensetzungen können - je nach Handhabung - als positiv und als negativ arbeitendes Photoresistsystem verwendet werden. Falls nach Belichtung einer entsprechenden Beschichtung direkt oder nach nur gelindem Erwärnen entwickelt wird, arbeitet das Resistsystem positiv. Die erfindungsgemässen Verbindungen wirken dann als Lösungsinhibitoren für die Beschichtung an den nicht belichteten Stellen. Die Verbindungen sind nämlich in wässrigen alkalischen Lösungen, wie sie für Positivresistsysteme üblicherweise als Entwickler verwendet werden, relativ schwer, nach der von der Bestrahlung herrührenden säurekatalysierten Spaltung der Etherbrücke zwischen Aromat und Pyranylgruppe dagegen leicht löslich. Werden die Resistsysteme aber nach der Belichtung auf über 100°C erhitzt, kann eine Umkehr der Tonalität erreicht werden, und die Systeme arbeiten nun negativ. Sie können aber ebenfalls mit den oben erwähnten wässerig alkalischen Entwicklern erhöhter Konzentration schwellungsfrei entwickelt werden, die nunmehr aber die unbelichteten Teile der Beschichtung besser lösen als die belichteten.

Die Erfindung betrifft daher auch ein Verfahren zur Erzeugung von positiven Abbildungen, enthaltend folgende Arbeitsschritte:
Beschichtung eines Substrates mit einer strahlungsempfindlichen Zusammensetzung wie oben definiert,
Belichtung des beschichteten Substrates in einem vorbestimmten Muster mit aktinischer Strahlung und,
gegebenenfalls nach kurzzeitiger Erwärmung auf eine Temperatur unter 100°C, Herauslösen der belichteten Bereiche der Beschichtung mit einem Entwickler.

Zur Durchfürhrung dieses Verfahrens stellt man im allgemeinen zunächst eine Lösung der erfindungsgemässen Zusammnsetzung her. Die Wahl des Lösungsmittels und die Konzentration richten sich hauptsächlich nach der Art der Zusammensetzung und nach dem Beschichtungsverfahren. Die Lösung wird mittels bekannten Beschichtungsverfahren auf ein Subsaat gleichfförmig aufgebracht, z.B. durch Schleudern, Tauchen, Rakelbeschichtung, Vorhanggiessverfahren, Aufpinseln, Sprühen und Reverse Rollbeschichtung. Es ist auch möglich, die lichtempfindliche Schicht auf einen temporären, flexiblen Träger zu bringen, und dann durch Schichtübertragung via Lamination das endgültige Subsaat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Dieser Schichtdickenbereich kann Werte von ca. 0,5 µm bis mehr als 100 µm umfassen.

Mögliche Einsatzgebiete der erfindungsgemässen Zusammensetzung sind die Verwendung als Photoresists für die Elektronik die Herstellung von Druckplatten, wie Offsetdruckplattern oder Siebdruckformen, der Einsatz beim Formteilätzen und insbesondere der Einsatz als Mikroresist bei der Herstellung integrierter Schaltkreise. Entsprechend unterschiedlich sind die Schichtträger und Verarbeitungsbedingungen.

Beim Einsatz als Mikroresist für integrierte und hochintegrierte Schaltkreise beispielsweise liegen die Schichtdicken zwischen 0,5 und 10 µm, bevorzugt zwischen 0,5 und 5 µm, insbesondere zwischen 0,5 und 1,5 µm.

Nach dem Beschichten wird das Lösungsmittel in der Regel durch Trocknen entfernt und es resultiert eine Schicht des Photoresists auf dem Träger. Die Beschichtungen weisen besonders schöne und glatte Oberflächen ohne Risse auf.

Nach der in üblicher Weise erfolgten bildmässigen Belichtung des Materials kann das beschichtete Substrat direkt in einem Entwickler entwickelt werden, oder gegebenenfalls kurzzeitig, z.B. bis zu etwa 5 Minuten erwärmt werden (sogenannter Post Exposure Bake), was zu einer Erhöhung der Lösungsrate des belichteten Resistmaterials führen kann. Die Temperatur muss dabei unter 100°C bleiben, sollte aber bevorzugt 60°C nicht überschreiten.

Die belichteten Stellen des Photolacks werden danach in einem Entwickler gelöst Die Wahl des jeweiligen Entwicklers richtet sich nach der Art des Photolacks, insbesondere nach der Natur des verwendeten Bindemittels oder der entstehenden Photolyseprodukte. Der Entwickler kann wässrige Lösungen von Basen umfassen, denen gegebenenfalls organische Lösungsmittel oder deren Mischungen zugesetzt wurden. Bevorzugt wird die Verwendung einer wässrigen Lösung einer Base als Entwicklungsmittel.

Geeignet als Entwickler zur Erzeugung positiver Strukturen sind z.B. wässrige alkalische Lösungen, wie sie auch für die Entwicklung von Naphthochinondiazidschichten eingesetzt werden. Dazu zählen insbesondere wässrige Lösungen von Alkalimetallsilikaten, -phosphaten und -hydroxiden.

Ein besonderer Vorteil der positiv arbeitenden Photoresists nach der Erfindung ist, dass sie auch eine hervorragende Inhibierung in metallionenfreien Entwicklern, sogenannten MIF-Entwicklern, zeigen. Dabei handelt es sich z.B. um wässrige Lösungen von Tetraalkylammoniumhydroxiden, beispielsweise von N(CH₃)₄OH oder N(C₄H₉)₄OH, die bei der Herstellung von integrierten Schaltkreisen zur Vermeidung von Metallkontaminationen vielfach gewünscht werden.

Die wässrigen Entwicklerlösungen können gegebenenfalls auch kleinere Mengen an Netzmitteln und/oder organischen Lösungsmitteln enthalten.
Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.
Ein typisches wässrig/organisches Entwicklungssystem basiert auf Butylcellosolve®/ Wasser.

Der Begriff'Belichtung in einem vorbestimmten Muster mit aktinischer Strahlung' beinhaltet sowohl die Belichtung durch eine Photomaske, die ein vorbestimmtes Muster enthält, beispielsweise ein Diapositiv, sowie die Belichtung durch einen Laserstrahl, der beispielsweise computergesteuert über die Oberfläche des beschichteten Substrates bewegt wird, und auf diese Weise ein Bild erzeugt.

Die Lichtempfindlichkeit der erfindungsgemässen Zusammensetzungen reicht in der Regel vom UV-Gebiet (ca. 200 nm) bis ca. 600 nm und umspannt somit einen sehr breiten Bereich, so dass als Lichtquellen an sich eine grosse Anzahl der verschiedensten Typen zur Anwendung kommen können. Es sind sowohl Punktlichtquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Excimer-Laser, wie z. B. Kryptonfluorid-Excimer-Laser, Fluoreszenzlampen, Argonglühlampen, Elektronenblitzlampen und photographische Flutlichtlampen. Der Abstand zwischen Lampe und erfindungsgemässem Bildmaterial kann je nach Anwendungszweck und Lampentyp bzw. -stärke variieren, z.B. zwischen 2 cm bis 150 cm. Geeignet sind auch andere Laserlichtquellen, z.B. Argonionen- oder Kryptonionenlaser. Bei dieser Art der Belichtung ist eine Photomaske im Kontakt mit der Photopolymerschicht nicht mehr unbedingt nötig; der gesteuerte Laser-Strahl kann direkt auf die Schicht schreiben. Hierzu ist die hohe Empfindlichkeit der erfindungsgemässen Materialien sehr vorteilhaft, die hohe Schreibgeschwindigkeiten bei relativ niedrigen Intensitäten erlaubt.

Die lichtempfindlichen Zusammensetzungen können gegebenenfalls auch Sensibilisatoren enthalten, um die spektrale Empfindlichkeit in einem bestimmten Wellenlängenbereich zu erhöhen. Zu diesen Sensibilisatoren zählen beispielsweise Michlers Keton, Benzophenone, Thioxanthone oder aromatische Kohlenwasserstoffe wie Anthracen, substituierte Anthracene, Pyren oder Perylen.

Besonders vorteilhaft lassen sich positiv arbeitende Photoresistbeschichtungen nach der Erfindung im UV-Bereich, insbesondere im Tief-UV-Bereich einsetzen. Dabei wird mit Strahlung einer Wellenlänge unterhalb etwa 300 nm belichtet, wie sie z.B. aus dem Licht von Quecksilberdampflampen herausgefiltert oder mittels Excimer-Laser erzeugt werden kann.

Besonders geeignet sind hierbei Photoresistzusammensetzungen auf Basis von Poly-(4-hydroxystyrolen) als Bindemittel. Poly-(4-hydroxystyrol) ist nämlich ein Harz, welches im Tief-UV-Bereich, verglichen z. B. mit Novolaken, nur äusserst schwach absorbiert. Die genannten Zusammensetzungen führen daher nach der bildmässigen Belichtung und Entwicklung zu Resiststrukturen, deren Profile wie gewünscht praktisch senkrecht sind. Absorbiert dagegen das Bindemittel einer Photoresistbeschichtung in dem Wellenlängenbereich, der zur Belichtung verwendet wird, so hat dies zur Folge, dass das eingestrahlte Licht besonders stark in den oberen Schichten des Resistfilms absorbiert wird und nur ein verhälnismässig geringer Teil in die tiefere Resistschicht vordringen und dort wirksam werden kann. Dies wiederum bewirkt, dass die Profile der Resiststrukturen nicht annähernd vertikal sind, sondern mit zunehmender Tiefe an Dicke zunehmen. Andererseits hat Poly-(4-hydroxystyrol) aber die Eigenschaft, dass es sich im Vergleich zu Novolaken sehr gut in den in der Praxis üblicherweise verwendeten wässrig-alkalischen Entwicklern löst. Bisher konnte es daher nur schlecht als Bindemittel in Photoresists für diese Art von Entwicklern eingesetzt werden, da keine Lösungsinhibitoren zur Verfügung standen, die einen genügend grossen Löslichkeitsunterschied zwischen den belichteten und nicht belichteten Teilen der Beschichtung erzeugen konnten. Bei Verwendung erfindungsgemässer Verbindungen als Lösungsinhibitoren kann nun die bessere Löslichkeit von Poly-(4-hydroxystyrol) gegenüber Novolaken sehr leicht ausgeglichen werden, indem nämlich erfindungsgemässe Verbindungen mit solchen Tetrahydropyranyloxysubstituenten eingesetzt werden, die relativ unpolare Reste aufweisen, insbesondere unpolare Reste R₃, etwa i-Butyl oder Phenyl.

Da die erfindungsgemässen Lösungsinhibitorverbindungen auf dem Prinzip der chemischen Amplifikation beruhen, sind diese Photoresists sehr empfindlich. Dies ist besonders deshalb günstig, weil viele Strahlungsquellen nur eine sehr schwache Lichtleistung unterhalb 300 nm aufweisen. Die erfindungsgemässen Photoresistzusammensetzungen werden bevorzugt mit Strahlungsdosen zwischen 50 mJ/cm und 5 mJ/cm belichtet, wobei günstige Belichtungszeiten möglich sind. Auch noch geringere Strahlungsdosen als die vorgenannten sind gegebenenfalls möglich.

Bei Belichtung mit Strahlung aus dem Tief-UV-Bereich können bei Einsatz erfindungsgemässer Photoresists Strukturelemente mit Abmessungen im Submikr-onbereich, beispielsweise zwischen 0,5 und 1 µm, ohne weiteres abgebildet werden. Diese Arbeitsweise ist daher besonders für die Herstellung hochintegrierter Schaltkreise geeignet. Die nach der anschliessenden Entwicklung ungelöst verbleibenden Bereiche weisen annähernd senkrechte Seitenwände auf.

Das Kontrastverhalten der Resists ist allgemein gut, ganz besonders wenn erfindungsgemässe Verbindungen der Formel I mit einem Kohlenwassertoffrest R₃ als Lösungsinhibitoren verwendet werden, der ebenfalls säurekatalysiert relativ leicht abspaltbar ist, z.B. ein allylisch ungesättigter Kohlenwasserstoff. Die Resists weisen auch einen sehr geringen Dunkelabtrag auf.

Ein weiterer Vorteil der erfinndungsgemässen Photoresistzusmmaensetzungen ist, dass sie bei Erzeugung positiver Abbildungen zwischen Belichtung und Entwicklung nicht unbedingt der oben schon erwähnten Erwärmungsbehandlung (Post-Exposure-Bake) unterzogen werden müssen, um die belichteten Stellen genügend löslich zu machen. Ein gemäss Stand der Technick bei Verwendung von Positivphotoresistsystemen, die nach dem Prinzip der chemischen Amplikation arbeiten, nötiger Verfahrensschritt kann somit vermieden werden.

Die Erfindung betrifft ferner ein Verfahren zum Erzeugen von negativen Abbildungen, das folgende Schritte enthält:
Beschichtung eines Substrats mit einer Zusammensetzung, wie sie oben beschrieben wurde,
Belichtung des beschichteten Substrats in einem vorbestimmten Muster mit aktinischer Strahlung und nachfolgende Erwärmung auf eine Temperatur zwischen 100°C und 170°C sowie Herauslösen der unbelichteten Bereiche der Beschichtung mit einem Entwickler.

Beschichtung und Belichtung können in der gleichen Weise erfolgen, wie es oben für das Verfahren zur Erzeugung positiver Abbildungen erläutert wurde. Zwischen Belichtung und Entwicklung muss die Resistschicht nunmehr jedoch für vorzugsweise etwa 5 bis 120 Sekunden aufeine Temperatur von 100-170°C erhitzt werden. Durch diese Erhitzung werden die belichteten Bereiche des Resists chemisch so verändert, dass das Resistmaterial an diesen Stellen in den üblichen, oben bereits genannten alkalisch-wässrigen Entwicklerlösungen für Positivphotoresists selbst dann praktisch unlöslich ist, wenn Entwicklerlösungen mit sehr hohen Konzentrationen verwendet werden. Auch Konzentrationen, die bereits zur Auflösung von unbelichtetem Resistmaterial führen, greifen es an den belichteten Stellen nicht mehr an, so dass der unbelichtete Resist weggelöst werden kann, d.h. eine negative Abbildung entsteht.

Wie bereits erwähnt, müssen in dem erfindungsgemässen Verfahren zur Erzeugung negativer Abbildungen die für Positivresist geeigneten Entwickler in höheren Konzentrationen verwendet werden, als für entsprechende positiv arbeitende Photoresistschicht geeignet wären, bei denen die unbelichteten Bereiche ja nicht aufgelöst werden dürfen. Geeignete Konzentrationen kann der Fachmann durch ein, zwei Orientierungsversuche leicht festlegen. Vielfach eignen sich kommerziell erhältliche Entwickler für Positivphotoresists, wenn sie unverdünnt eingesetzt werden. Im allgemeinen tritt bei der Entwicklung keine Schwellung der Resistschicht auf.

Ein weiterer Gegenstand der Erfindung sind die unter Verwendung der oben beschriebenen Verfahren herstellbaren Erzeugnisse, insbesondere die, die künstlich erzeugte Strukturelemente mit Abmessungen von weniger als 1 µm aufweisen.

Beispiel 1: Herstellung von Bis-2-(6-ethoxytetrahydropyranylether) von 4,4'-Isopropylidendiphenol (A)

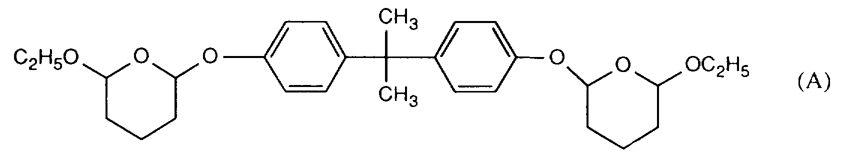

In einem 150 ml 3-Hals-Rundkolben mit Magnetrührer, Innenthermometer, Tropftrichter und Stickstoffüberleitung werden bei 5°C zu einer Mischung aus 8,9 g (39 mMol) 4,4'-Isopropylidendiphenol (Bisphenol A) und 0,18 g p-Toluolsulfonsäure in 100 ml Ether langsam 10,0 g (78 mMol) 2-Ethoxy-3,4-dihydro-2H-pyran (der Firma Aldrich) getropft. Nach 30 Minuten wird die Kühlung entfernt. Danach lässt man während 15 h bei 20°C ausreagieren. Das Reaktionsgemisch wird mit 0,5 N NaOH-Lösung neutralisiert, und die vereinten Etherextrakte werden dann dreimal mit Wasser gewaschen. Die Etherphase wird mit Magensiumsulfat getrocknet und eingeengt. Es werden 18,7 g (98 %) NMR-reines Produkt als viskoses, klares Oel erhalten.

NMR-Daten (100 MHz, CDCl₃): 7,15 (d, 4H); 6,99 (d, 4H); 5,63 (m, 2H); 4,91 (m, 2H); 4,0-3,3 (m, 4H); 2,1-1,85 (m, 12H); 1,63 (s, 6H); 1,15 (t, 6H).

Beispiel 2: Eine Resistlösung wird durch Auflösung von 25 Teilen des Bis-2-(6-ethoxytetrahydropyranylether) von 4,4'-Isopropylidendiphenol aus Beispiel 1, 75 Teilen eines Polyparahydroxystyrols (Resin M® der Firma Maruzen Oil) und 5 Teilen Triphenylsulfoniumhexafluoroarsenat in 250 Teilen Cyclohexanon bereitgestellt.

So hergestellte Resistlösung wird bei 2500 U/min auf 4-Zoll-Siliciumwafer aufgeschleudert und bei 120°C während 2 Minuten getrocknet, wobei homogene Filme mit einer Schichtdicke von 0,75 µm resultieren.

Die Filme werden durch eine Chrom-Quarz-Maske mit kleinsten Strukturen von 0,5 µm mit Licht der Wellenlänge 254 nm (Interferenzfilter, 100 nm Bandbreite) kontaktbelichtet. Als Lichtquelle dient eine Hg-Dampflampe; die eingestrahlte Dosis beträgt 3 mJ/cm.

Direkt anschliessend wird mit einer 0,2 N NaOH-Lösung entwickelt. Es werden 0,5 µm l/s (= lines and spaces ≙ nebeneinanderliegenden, gleich breiten belichteten und unbelichteten Bereichen) bei annähernd vertikalen Wandprofilen gut aufgelöst.

Beispiel 3: Herstellung von Bis-2-(6-isobutoxytetrahydropyranylether) von 9,9-Bis-(4-hydroxyphenyl)-fluoren (B)

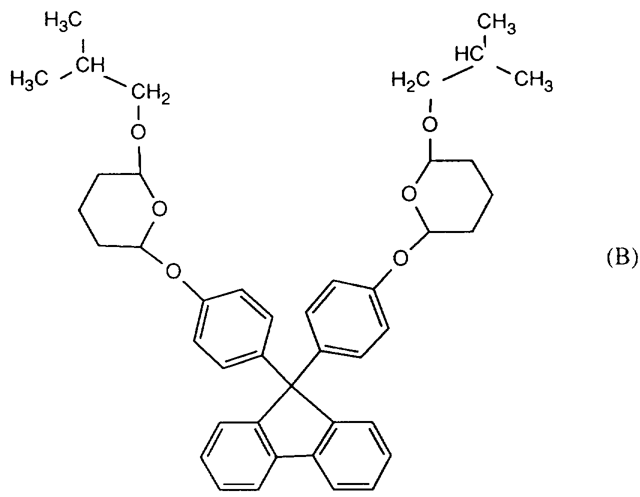

In einem 250 ml 3-Halsrundkolben mit Magnetrührer, Innenthermometer, Tropftrichter und Stickstoffüberleitung werden bei 20°C zu 8,8 g (25 mMol) 9,9-Bis(4-hydroxyphenyl)-fluoren und 0,1 g p-Toluolsulfonsäure in 200 ml Diethylether 8,6 g (55 mMol) 2-Isobutoxy-3,4-dihydro-2H-pyran langsam zugetropft. Man lässt während 20 h ausreagieren und versetzt danach mit 100 ml 1 N NaOH-Lösung. Die organische Phase wird zweimal mit Wasser gewaschen, über Magnesiumsulfat getrocknet und eingeengt. Dabei resultieren 14 g (21 mMol, 84 %) des gewünschten Produkts als farbloses Pulver, welches durch Umkristallisation in n-Hexan weiter gereinigt werden kann. Schmelzpunkt (unkorrigiert): 83°C
¹H-NMR (100 MHz): 7,6-6,6 (m, 16H); 5,6 (m, 2H); 4,8 (m, 2H); 3,4 (dd, 2H); 3,0 (dd, 2H); 1,9-1,5 (m, 14H); 0,8 (d, 12H)
TGA (10°/min): -5 % bei 233°C; -37 % bei 290°C

Beispiel 4: Eine Resistlösung wird durch Auflösen von 25 Teilen des Bis-2-(6-isobutoxytetrahydropyranylether) von 9,9-Bis(4-hydroxyphenyl)-fluoren aus Beispiel 3, 75 Teilen Poly-para-hydroxystyrol (Resin-M®) und 5 Teilen Triphenylsulfonium-(trifluoromethansulfonat), im folgenden auch Triphenylsulfoniumtriflat genannt, in 250 Teilen Cyclopentanon bereitgestellt. So hergestellte Resistlösung wird bei 4000 U/min auf 4-Zoll-Siliziumwafer aufgeschleudert und bei 120°C während 2 Minuten getrocknet, wobei homogene Filme einer Schichtdicke von 1,0 µm resultieren.
Anschliessend werden die Filme durch eine Chrom-Quarz-Maske mit kleinsten Strukturen von 0,5 µm kontaktbelichtet mit einer Dosis von 20 mJ/cm. Direkt anschliessend wird mit einer 0,17 N NaOH-Lösung entwickelt. Dabei gelingt die bildmässige Erzeugung von positiven Submikronstrukturen.

Beispiel 5: Beschichtete 4-Zoll-Siliziumwafer aus Beispiel 4 werden durch eine Chrom-Quarz-Maske belichtet, wobei die Dosis 5 mJ/cm beträgt. Anschliessend wird das Material während 30 Sekunden auf 130°C erhitzt und danach in einer 2,38 %-igen Lösung von Tetramethylammoniumhydroxid entwickelt, wobei die unbelichteten Stellen herausgelöst werden. Das Material zeigt während dem Entwicklungsprozess keine Aufschwellung und erlaubt die Erzeugung negativer Abbildungen mit kleinsten Strukturen von 0,5 µn l/s.

Beispiel 6: Herstellung von Bis-2-(6-ethoxytetrahydropyranylether) von 9,9-Bis-(4-hydroxyphenyl)-fluoren (C) 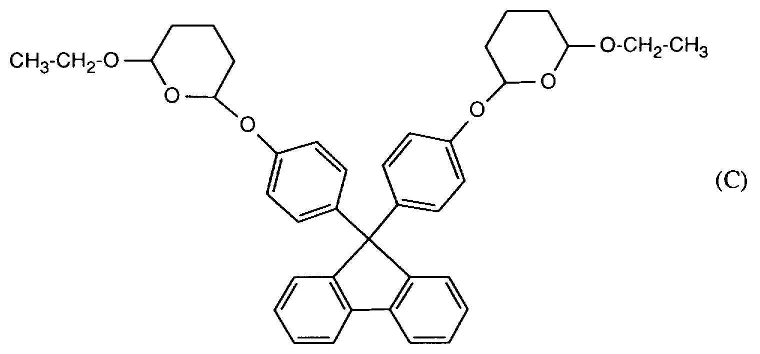 In einem 250 ml 3-Halsrundkolben mit Magnetrührer, Innenthermometer, Tropftrichter und Stickstoffüberleitung werden bei 20°C zu 8,8 g (25 mMole) 9,9-Bis-(4-hydroxyphenyl)-fluoren und 0,1 g p-Toluolsulfonsäure in 200 ml Diethylether langsam 7,0 g (55 mMol) 2-Ethoxy-3,4-dihydro-2H-pyran zugetropft. Man lässt während 20 h bei 20°C ausreagieren und versetzt danach mit 100 ml 1 N NaOH-Lösung. Die resultierende organische Phase wird zweimal mit Wasser gewaschen, über Magnesiumsulfat getrocknet und eingeengt, wobei 10 g (17 mMol; 68 %) des gewünschten Produkts als weisse Masse anfallen, welche durch Umkristallisation aus n-Hexan weiter gereinigt werden kann. Schmelzpunkt (unkorrigiert): 110°C
¹H-NMR (100 MHz): 7,8-6,6 (m, 16H); 5,6 (m, 2H); 4,8 (m, 2H); 3,7 (q, 2H); 3,4 (q, 2H); 2,0-1,6(m, 12H); 1,1(t,6H)
TGA (10°/min): -5 % bei 237°C; -33 % bei 300°C

Beispiel 7: Eine Resistlösung wird analog zu Beispiel 4 durch Auflösung von 25 Teilen Tetrahydropyranylether aus Beispiel 6, 75 Teilen Resin-M® und 5 Teilen Triphenylsulfoniumtriflat in 250 Teilen Cyclopentanon bereitgestellt und auf einen Siliziumwafer aufgeschleudert. Nach dem Trocknen bei 120°C während 2 Minuten resultieren homogene Resistfilme einer Schichtdicke von 0,9 µm. Bildmässiges Bestrahlen mit einer Dosis von 20 mJ/cm bei 254 nm und anschliessende Entwicklung gemäss Beispiel 2 liefert originalgetreue positive Abbildungsstrukturen.

Beispiel 8: Herstellung von Bis-2-(6-methoxytetrahydropyranylether) von 1,1-Di-4-hydroxyphenyl-1-phenylethan (D) 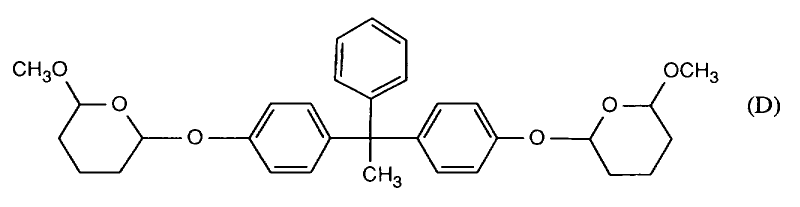 In einem 100 ml 3-Halsrundkolben mit Magnetrührer, Innenthermometer, Tropftrichter und Stickstoffüberleitung werden bei 20°C zu 7,5 g (26 mMol) Bisphenol-C (1,1-Di-4-hydroxyphenyl-1-phenylethan) und 0,15 g p-Toluolsulfonsäure in 40 ml Diethylether langsam 6,5 g (57 mMol) 2-Methoxy-3,4-dihydro-2H-pyran zugetropft. Man lässt während 20 h bei 20°C weiterreagieren und versetzt danach mit 100 ml 1 N NaOH-Lösung. Die organische Phase wird 2x mit Wasser gewaschen, über Magnesiumsulfat getrocknet und eingeengt, wobei 7,3 g (14 mMol, 54 %) des gewünschten Produkts als weisses Pulver resultieren, welches durch Umkristallisation aus n-Hexan weiter gereinigt werden kann.
Schmelzpunkt (unkorrigiert): 60°C
¹H-NMR (100 MHz): 7,4-6,7 (m, 13H); 5,6 (m, 2H); 4,8 (m, 2H); 3,4 (s, H); 2,2-1,6 (m, 12H)
Elem. Analyse:
C: ber. 74,11 %; gef. 74,97 %
H: ber. 7,39 %; gef. 7,13 %

Beispiel 9: Eine Resistlösung wird analog zu Beispiel 4 durch Auflösung von 25 Teilen des Bis-2-(6-methoxytetrahydropyranylether) von 1,1-Di-4-hydroxyphenyl-1-phenylethan aus Beispiel 8, 75 Teilen Resin-M® und 5 Teilen Triphenylsulfoniumtriflat in 250 Teilen Cyclopentanon bereitgestellt und auf einen Siliziumwafer bzw. eine 1-Zoll-Quarzscheibe aufgeschleudert. Nach dem Trocknen bei 20°C während 2 Minuten resultieren Resistfilme mit einer Schichtdicke von 1,2 µm, deren optische Dichte (Quarzscheibe) 0,51 beträgt. Nach der bildmässigen Belichtung analog Beispiel 2 oder Beispiel 4 wird in 0,17 N NaOH-Lösung entwickelt wobei positive Strukturen im Submikrobereich aufgelöst werden können.

Beispiel 10: In einem Druckreaktor lässt man 45 g (375 mMol) Phenylvinylether, 21 g (375 mMol) Acrolein und 0,66 g (6 mMol) Hydrochinon während 1 h bei 185°C reagieren. Nach dem Abkühlen wird 2-Phenoxy-3,4-dihydro-2H-pyran durch Destillation bei 80°C/0,0267 kPa in einer Ausbeute von 30,2 g (172 mMol; 46 %) als klare Flüssigkeit isoliert. Siedepunkt: 80°C/0,0267 kPa
¹H-NMR (100 MHz): 7,26 (m, 2H); 7,1-6,9 (m, 3H); 6,22 (d, 1H); 5,68 (m, 1H); 4,83 (m, 1H); 2,4-2,2 (m, 1H); 1,85-2,2 (m, 3H)

Beispiel 11: Herstellung von Bis-2-(6-phenoxytetrahydropyranylether) von 4,4'-Isopropylidendiphenol (E)

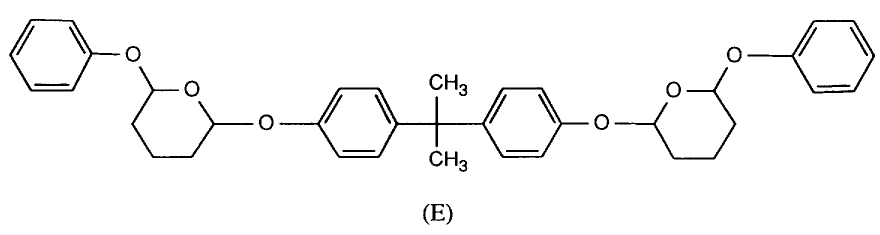

In einem 100 ml 3-Halsrundkolben mit Magnetrührer, Innenthermometer, Tropftrichter und Stickstoffüberleitung werden bei 10°C zu 2,3 g (10 mMol) 4,4'-Isopopylidendiphenol (Bisphenol-A) und 50 mg p-Toluolsulfonsäure in 30 ml Diethylether langsam 3,9 g (22 mMol) 2-Phenoxy-3,4-dihydro-2H-pyran zugetropft. Man lässt auf Raumtemperatur kommen und danach während 30 h ausreagieren. Schliesslich wird mit 50 ml 1 N NaOH-Lösung versetzt, die organische Phase wird danach zweimal mit Wasser gewaschen, über Magnesiumsulfat getrocknet und eingeeingt wobei 4,7 g (8,1 mMol; 81 %) der gewünschten Substanz als farbloses viskoses Oel anfallen. Das Produkt kann durch Umkristallisation aus Methanol weitergereinigt werden.
Schmelzpunkt (unkorrigiert): 54°C
¹H-NMR (100 MHz): 7,3-6,7 (m, 18H); 5,7 (m, 4H); 2,2-1,7 (m, 12H); 1,6 (m, 6H)
TGA (10°/min): -5 % bei 225°C; -47 % bei 293°C

Beispiel 12: Durch Auflösen von 25 Teilen Bis-2-(6-phenoxytetrahydropyranylether) von 4,4'-Isopropylidendiphenol aus Beispiel 10, 75 Teilen Resin-M® und 5 Teilen Triphenylsulfoniumtriflat in 250 Teilen Cyclopentanon wird eine Resistlösung bereitgestellt, welche bei 3500 U/min auf einen 4-Zoll-Siliziumwafer bzw. eine 1-Zoll-Quarzscheibe aufgeschleudert wird. Nach der Trocknung des Materials bei 120°C während 2 Minuten resultieren Resistfilme einer Schichtdicke von 1,1 µm, die bei 254 nm eine optische Dichte von 0,48 aufweisen.

Der beschichtete Siliziumwafer wird danach durch eine Quarzmaske, deren kleinste Strukturen 0,5 µm betragen, bildmässig belichtet. Alss Lichtquelle dient eine Quecksilberdampflampe, in deren Strahlengang ein 254 nm-Interferenzfilter mit 10 nm Bandbreite eingebaut ist. Die Dosis beträgt dabei 20 mJ/cm. Direkt nach der Belichtung wird in einem Entwickler auf Tetramethylammoniumhydroxidbasis entwickelt, wobei ein originalgetreues positives Abbild der Maskensaukturen erhalten wird.

## Patentansprüche

1. Nichtpolymere Verbindungen, wobei dieser Begriff auch Oligomere eines Polymerisationsgrades unter 10 umfasst und die Verbindungen mindestens ein aromatisches Ringsystem mit einem oder mehreren Tetrahydropyranyloxysubstituenten der Formel I aufweisen und wobei die Verbindungen insgesamt mindestens zwei dieser Substituenten enthalten: 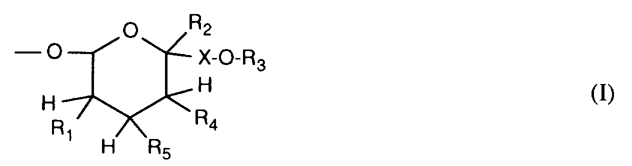 wobei
R₁ Wasserstoff, Halogen, Alkyl, Cycloalkyl, Aryl, Alkoxy oder Aryloxy,
R₂ Wasserstoff, Alkyl, Cycloalkyl oder Aryl,
R₃ einen gesättigten oder ungesättigten Kohlenwasserstoffrest,
R₄, R₅ unabhängig voneinander Wasserstoff, Halogen, Alkyl, Alkoxy oder Aryloxy und
X eine direkte Einfachbindung, eine Methylen- oder Ethylenbrücke bedeuten können.

2. Verbindungen nach Anspruch 1, wobei in der Formel I
R₁ Wasserstoff, Halogen, C₁-C₅-Alkyl, Phenyl, substituiertes Phenyl, C₁-C₅-Alkoxy, Phenoxy oder substituiertes Phenoxy,
R₂ Wasserstoff, C₁-C₅-Alkyl oder Phenyl,
R₃ einen gesättigten oder ungesättigten Kohlenwasserstoffrest mit 1 bis 20 Kohlenstoffatomen und
R₄, R₅ unabhängig voneinander Wasserstoff, Halogen, C₁-C₅-Alkyl, C₁-C₅-Alkoxy, Phenoxy oder substituiertes Phenoxy bedeuten können.

3. Verbindungen nach Anspruch 2, wobei in der Formel I
R₁ Wasserstoff, Methyl oder Phenyl,
R₂, R₄, R₅ alle Wasserstoff und
R₃ einen gesättigten oder ungesättigten acyclischen Kohlenwasserstoffrest mit 1 bis 8 Kohlenstoffatomen oder Phenyl bedeuten können.

4. Verbindungen nach einem der Ansprüche 1 bis 3, wobei in der Formel I X eine direkte Einfachbindung bedeutet.

5. Verbindungen nach einem der Ansprüche 1 bis 4,
die maximal 100 Gerüstatome aufweisen, wobei als Gerüstatome alle Kohlenstoff- und Heteroatome gezählt werden, jedoch die Tetrahydropyranyloxygruppen der Formel I nicht mitgerechnet sind;
bei denen mindestens 75% der Gerüstatome zu aromatischen Systemen gehören; die mindestens zwei Tetrahydropyranyloxysubstituenten der Formel I aufweisen und bei denen die Gesamtzahl der Substituenten der Formel I mindestens so gross ist, dass auf zwei aromatische Ringe in den Verbindungen mindestens ein Substituent der Formel I kommt, wobei mit aromatischen Ringen jeweils die einzelnen aromatischen Ringe eines aromatischen Systems gemeint sind.

6. Verbindungen nach einem der Ansprüche 1 bis 5, die als aromatische Ringsysteme lediglich nicht konjugierte Systeme mit 6 Ringkohlenstoffatomen aufweisen.

7. Die Verbindungen nach Anspruch 6 mit der Formel II oder III 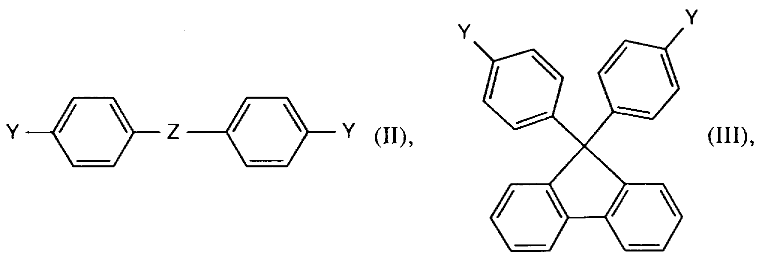 wobei
Y jeweils einen Tetrahydropyranyloxysubstituenten der Formel 1 und
Z entweder eine direkte Einfachbindung oder eine der folgenden Gruppen bedeuten kann: -S-; -O-; -SO-; -SO₂-; -CO- oder -C(R₆)(R₇)-, wobei R₆ Wasserstoff, Methyl oder Aryl sowie R₇ Wasserstoff oder Methyl darstellen können.

8. Zusammensetzung, enthaltend eine nichtpolymere Verbindung, wobei dieser Begriff auch Oligomere eines Polymerisationsgrades unter 10 umfasst und die Verbindung mindestens ein aromatisches Ringsystem mit einem oder mehreren Tetrahydropyranyloxysubstituenten der Formel I gemäss Anspruch 1 aufweist, und eine Verbindung, die unter Einwirkung aktinischer Strahlung Säure bildet, sowie ein Bindemittel.

9. Verfahren zum Erzeugen von positiven Abbildungen, enthaltend folgende Schritte: Beschichtung eines Substrates mit einer Zusammensetzung gemäss Anspruch 8, Belichtung des beschichteten Substrats in einem vorbestimmten Muster mit aktinischer Strahlung und,
gegebenenfalls nach kurzzeitiger Erwärmung auf eine Temperatur unter 100°C, Herauslösen der belichteten Bereiche der Beschichtung mit einem Entwickler.

10. Verfahren zum Erzeugen von negativen Abbildungen, enthaltend folgende Schritte: Beschichtung eines Substrates mit einer Zusammensetzung gemäss Anspruch 8, Belichtung des beschichteten Substrats in einem vorbestimmten Muster mit aktinischer Strahlung und nachfolgende Erwärmung auf eine Temperatur zwischen 100 und 170°C sowie
Herauslösen der unbelichteten Bereiche der Beschichtung mit einem Entwickler.

11. Verfahren nach Anspruch 9 oder 10, bei dem aktinische Strahlung mit einer Wellenlänge unter 300 nm zur Belichtung verwendet wird.

12. Verfahren nach einem der Ansprüche 9 bis 11, bei dem das beschichtete Substrat mit Strahlung in einem Muster belichtet wird, das Strukturelemente mit Abmessungen von weniger als 1 µm enthält.

13. Verfahren nach einem der Ansprüche 9, 11 oder 12, bei dem das beschichtete Substrat zwischen Belichtung und Entwicklung keiner Erwärmungsbehandlung mehr unterzogen wird.

14. Die unter Verwendung eines Verfahrens nach einem der Ansprüche 9 bis 13 herstellbaren Erzeugnisse.

15. Erzeugnisse nach Anspruch 14, die künstlich erzeugte Strukturelemente mit Abmessungen von weniger als 1 µm aufweisen.

## Claims

1. A non-polymeric compound, this term also including oligomers with a degree of polymerisation of below 10, which contains at least one aromatic ring system carrying one or more tetrahydropyranyloxy substituents of formula I and containing overall at least two of these substituents: 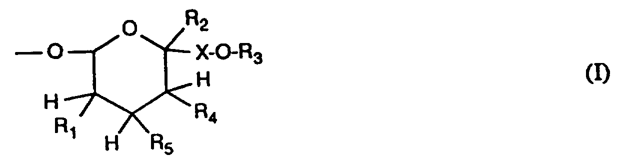 wherein
R₁ can be hydrogen, halogen, alkyl, cycloalkyl, aryl, alkoxy or aryloxy,
R₂ can be hydrogen, alkyl, cycloalkyl or aryl,
R₃ can be saturated or unsaturated hydrocarbon radical,
R₄ and R₅ can each independently of the other be hydrogen, halogen, alkyl, alkoxy or aryloxy,
and
X can be a direct single bond or a methylene or ethylene bridge.

2. A compound according to claim 1, wherein in the formula I
R₁ can be hydrogen, halogen, C₁-C₅alkyl, phenyl, substituted phenyl, C₁-C₅alkoxy, phenoxy or substituted phenoxy,
R₂ can be hydrogen, C₁-C₅alkyl or phenyl,
R₃ is a saturated or unsaturated hydrocarbon radical of 1 to 20 carbon atoms, and
R₄ and R₅ can each independently of the other be hydrogen, halogen, C₁-C₅alkyl, C₁-C₅alkoxy, phenoxy or substituted phenoxy.

3. A compound according to claim 2, wherein in the formula I
R₁ can be hydrogen, methyl or phenyl,
R₂, R₄ and R₅ can all be hydrogen, and
R₃ can be a saturated or unsaturated acyclic hydrocarbon radical of 1 to 8 carbon atoms or phenyl.

4. A compound according to one of claims 1 to 3, wherein in the formula I X is a direct single bond.

5. A compound according to one of claims 1 to 4, which contains a maximum of 100 atoms in the molecule, including all carbon and hetero atoms but not the tetrahydropyranyloxy groups of formula I, at least 75% of which atoms belong to aromatic systems, said compound containing at least two tetrahydropyranyloxy substituents of formula I and the total number of substituents of formula I being at least so great that there is at least one substituent of formula I for every two aromatic rings in the compound, the expression "aromatic rings" being understood as meaning the individual aromatic rings of an aromatic system.

6. A compound according to one of claims 1 to 5, which contains as aromatic ring systems solely non-conjugated systems containing 6 ring carbon atoms.

7. A compound according to claim 6 of formula II or III 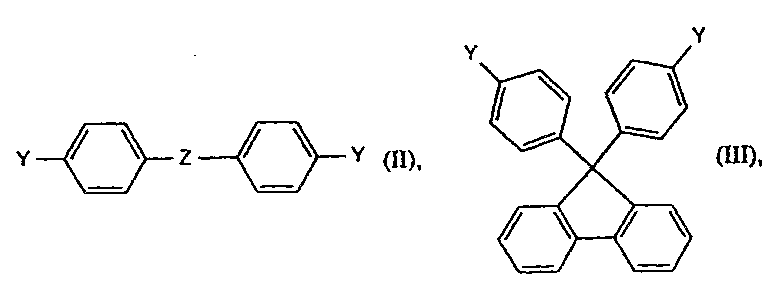 wherein each Y is a tetrahydropyranyloxy substituent of formula I and Z is either a direct single bond or can be one of the following groups: -S-; -O-; -SO-; -SO₂-; -CO- or -C(R₆)(R₇)-, where R₆ can be hydrogen, methyl or aryl and R₇ can be hydrogen or methyl.

8. A composition comprising a non-polymeric compound, this term also including oligomers with a degree of polymerisation of below 10, which contains at least one aromatic ring system carrying one or more tetrahydropyranyloxy substituents of formula I according to claim 1, a compound which generates acid when exposed to actinic radiation, and a binder.

9. A process for producing positive images, comprising the following steps: coating a substrate with a composition according to claim 8, exposing the coated substrate in a predetermined pattern to actinic radiation, and, after optional brief heating to a temperature below 100°C, dissolving out the exposed areas of the coating with a developer.

10. A process for the production of negative images, comprising the following steps:
coating a substrate with a composition according to claim 8,
exposing the coated substrate in a predetermined pattern to actinic radiation and subsequently heating to a temperature between 100 and 170°C, and
dissolving out the unexposed areas of the coating with a developer.

11. A process according to claim 9 or 10, wherein actinic radiation with a wavelength below 300 nm is used for the exposure.

12. A process according to one of claims 9 to 11, wherein the coated substrate is exposed to radiation in a pattern which contains structural elements with dimensions of less than 1 µm.

13. A process according to one of claims 9, 11 or 12, wherein the coated substrate is no longer subjected to heat treatment between exposure and development.

14. A product preparable using a process according to one of claims 9 to 13.

15. A product according to claim 14, which has artificially produced structural elements with dimensions of less than 1 µm.

## Revendications

1. Composés non polymères, cette expression englobant aussi des oligomères ayant un degré de polymérisation inférieur à 10, les composés contenant au moins un système cyclique aromatique avec un ou plusieurs substituants tétrahydropyranyloxy de formule I et les composés contenant en tout au moins deux de ces substituants: 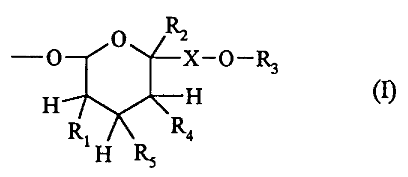 dans laquelle
R₁ peut représenter un atome d'hydrogène ou d'halogène ou un reste alkyle, cycloalkyle, aryle, alcoxy ou aryloxy;
R₂ peut représenter un atome d'hydrogène ou un reste alkyle, cycloalkyle ou aryle,
R₃ peut représenter un reste hydrocarboné saturé ou insaturé,
R₄ et R₅ peuvent représenter indépendamment l'un de l'autre un atome d'hydrogène ou d'halogène ou un reste alkyle, alcoxy ou aryloxy, et
X peut représenter une liaison simple directe ou un pont méthylène ou éthylène.

2. Composés selon la revendication 1, dans lesquels, dans la formule I,
R₁ peut représenter un atome d'hydrogène ou d'halogène ou un reste alkyle en C₁-C₅, phényle, phényle substitué, alcoxy en C₁-C₅, phénoxy ou phénoxy substitué,
R₂ peut représenter un atome d'hydrogène ou un reste alkyle en C₁-C₅ ou phényle,
R₃ peut être un reste hydrocarboné saturé ou insaturé de 1 à 20 atomes de carbone et
R₄, R₅ peuvent représenter, indépendamment l'un de l'autre, un atome d'hydrogène ou d'halogène ou un reste alkyle en C₁-C₅, alcoxy en C₁-C₅, phénoxy ou phénoxy substitué.

3. Composés selon la revendication 2, dans lesquels, dans la formule I
R₁ est un atome d'hydrogène ou un reste méthyle ou phényle,
R₂, R₄, R₅ sont tous des atomes d'hydrogène et
R₃ représente un reste hydrocarboné acyclique saturé ou insaturé de 1 à 8 atomes de carbone ou un reste phényle.

4. Composés selon l'une des revendications 1 à 3, dans lesquels, dans la formule I, X représente une liaison simple directe.

5. Composés selon l'une des revendications 1 à 4,
qui contiennent au maximum 100 atomes dans le squelette, si l'on compte comme atomes du squelette tous les atomes de carbone et les hétéroatomes, les groupes tétrahydropyranyloxy de formule I n'étant cependant pas inclus dans le compte;
dans lesquels au moins 75 % des atomes du squelette appartiennent à des systèmes aromatiques;
qui contiennent au moins deux substituants tétrahydropyranyloxy de formule I; et
dans lesquels le nombre total des substituants de formule I est au moins assez grand pour qu'il y ait au moins un substituant de formule I sur deux cycles aromatiques dans les composés, les cycles aromatiques signifiant les différents cycles aromatiques d'un système aromatique.

6. Composés selon l'une quelconque des revendications 1 à 5, qui présentent comme systèmes à cycles aromatiques, uniquement des systèmes non conjugués avec 6 atomes de carbone de cycle.

7. Composés selon la revendication 6, ayant la formule II ou III 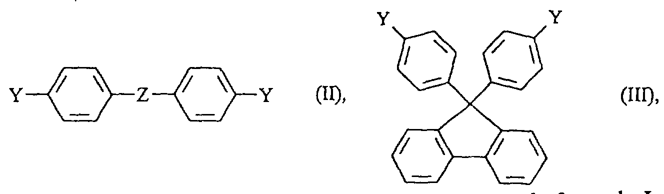 où Y est dans chaque cas un substituant tétrahydropyranyloxy de formule I et Z peut représenter soit une liaison simple directe, soit l'un des groupes suivants: -S-; -O-; -SO-; -SO₂-; -CO- ou -C(R₆)(R₇)-, où R₆ peut être un atome d'hydrogène ou un reste méthyle ou aryle et R₇ peut être un atome d'hydrogène ou un groupe méthyle.

8. Composition contenant un composé non polymère, cette expression englobant aussi des oligomères ayant un degré de polymérisation inférieur à 10, et le composé contenant au moins un système cyclique aromatique avec un ou plusieurs substituants tétrahydropyranyloxy de formule I selon la revendication 1, et un composé qui forme un acide sous l'effet d'un rayonnement actinique, ainsi qu'un liant.

9. Procédé de production d'images positives, comportant les étapes suivantes:
le revêtement d'un substrat à l'aide d'une composition selon la revendication 8,
l'exposition dans une configuration prédéterminée du substrat revêtu à un rayonnement actinique et,
éventuellement après un chauffage de courte durée à une température inférieure à 100°C, la dissolution des zones exposées du revêtement à l'aide d'un révélateur.

10. Procédé de production d'images négatives, comportant les étapes suivantes:
le revêtement d'un substrat à l'aide d'une composition selon la revendication 8,
l'exposition dans une configuration prédéterminée du substrat revêtu à un rayonnement actinique, suivie d'un chauffage à une température comprise entre 100°C et 170°C et
la dissolution des zones non exposées du revêtement avec un révélateur.

11. Procédé selon la revendication 9 ou 10, dans lequel on utilise pour l'exposition un rayonnement actinique ayant une longueur d'onde inférieure à 300 nm.

12. Procédé selon l'une des revendications 9 à 11, dans lequel on expose à un rayonnement le substrat revêtu dans une configuration qui contient des éléments de structure ayant des dimensions inférieures à 1 µm.

13. Procédé selon l'une des revendications 9, 11 ou 12, dans lequel on ne soumet le substrat revêtu à aucun traitement thermique entre l'exposition et le développement.

14. Produits pouvant être obtenus à l'aide d'un procédé selon l'une des revendications 9 à 13.

15. Produits selon la revendication 14, qui présentent des éléments de structure produits par synthèse ayant des dimensions inférieures à 1 µm.
